# EUROPEAN PATENT SPECIFICATION

(11) **EP 1 512 400 B1**
(45) Date of publication and mention of the grant of the patent: **10.03.2010**
(21) Application number: 04254982.4
(22) Date of filing: 19.08.2004
(51) Int. Cl.: A61K 31/415, A61P 25/00

(54) **Treatment of neurodegenerative diseases**
Behandlung von neurodegenerativen Erkrankungen
Traitement de maladies neurodégénératives

(30) Priority: 03.09.2003 US 653859
(43) Date of publication of application: 09.03.2005
(73) Proprietor: Carlsbad Technology Inc, Carlsbad, CA 92008 (US)
(72) Inventor: Fu, Wen-Mei, Da-an District, Taipei 06 (TW); Liou, Houng-Chin, Sinyi District, Taipei (TW); Lu, Dah-Yuu, Jhongjheng District, Taipei 06 (TW); Teng, Che-Ming, Taipei 106 (TW); Kuo, Sheng-Chu, Taichung (TW); Lee, Fang-Yu, Tachia Taichung (TW)
(74) Representative: den Hartog, Jeroen H.J.

(56) References cited:
- EP-A- 1 227 099
- WO-A-01/53268
- WO-A-03/068754
- US-A1- 2003 004 355
- US-A1- 2003 105 149
- G. GARTHWAITE AND ALL.: "Soluble guanylyl cyclase activator YC-1 protects white matter axons from nitric oxide toxicity and metabolic stress, probably through Na+ channel inhibition" MOLECULAR PHARMACOLOGY, vol. 61, no. 1, 2002, pages 97-104, XP002301498

## Description

### BACKGROUND

Neurodegenerative diseases, which represent a large group of neurological disorders, include Alzheimer's disease, Parkinson's disease, Huntington disease, glaucoma neurodegeneration and amyotrophic lateral sclerosis. These diseases are characterized by a progressive and specific loss of neurons. Patients with neurodegenerative diseases are likely to show localized to generalized atrophy of central and/or peripheral neurons, leading to compromises in both mental and physical functions. Mentally, patients exhibit forgetfulness, poor memory, reduced mental capacity, emotional disturbance, or unclear speech. Physically, patients exhibit partial to complete incontinence, aspiration of food particles, tremor, poor balance, muscle rigidity, or muscle paralysis.

WO 03/068754 and WO 01/53268 disclose various substituted indazoles, but not 3-furyl-indazoles, and their use for treatment of neurodegenerative diseases.

US 2003/105149 and WO 01/57024 disclose various substituted indazoles and their use for treating disorders associated with Huntington's disease, but not for treatment of Huntington's diseases as such.

### SUMMARY OF THE INVENTION

In one respect, this invention features use of a compound of formula (I) to prepare a therapeutic agent for treating Huntington's disease. The treatment includes administrating to a subject in need thereof a fused pyrazolyl compound of formula (I):

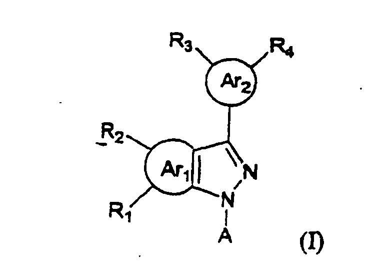

A is 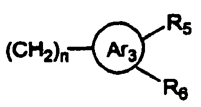 (referred to as "(CH₂)ₙAr₃(R₅)(R₆)" hereinafter); each of Ar₁, and Ar₃, is phenyl, Ar₂ is 5' furyl; each of R₁, R₂, R₃, R₄, R₅, and R₆, independently, is H, halogen, R, C(O)OH, C(O)OR, C(O)SH, C(O)SR, C(O)NH₂, C(O)NHR, C(O)NRR', ROH, ROR', RSH, RSR', NHR, NRR', RNHR'; or RNR'R", or R₁ and R₂ together, R₃ and R₄ together, or R₅ and R₆ together are ORO. Each of R, R', and R", independently is C₁∼C₆ alkyl; and n is 1, 2, or 3. The compound is in an effective amount for treating Huntington's disease.

Formula (I) includes a pyrazolyl core and at least two aryl groups, e.g., Ar₁, or Ar₂. The compounds of formula (I) are featured by that A is (CH₂)ₙAr₃(R₅)(R₆). In these compounds, Ar₁ is phenyl, and R₁ and R₂ are substituted at positions 4 and 5 of phenyl, respectively. Ar₂ is 5'-furyl, and one of R₃ and R₄ is substituted at position 2 of 5'-furyl. Ar₃ is phenyl.

Another subset of the compounds of formula (I) are featured by that A is H. In these compounds, Ar₁ is phenyl, and R₁ and R₂ are substituted at positions 4 and 5 of phenyl, respectively. Ar₂ is 5'-furyl, and one of R₃ and R₄ is substituted at position 2 of 5'-furyl.

The term "aryl" as used herein includes phenyl, thienyl, furyl, or pyrrolyl, each of which optionally includes one or more substituted moieties. The substituted moieties may be the same as or different from R₁, R₂, R₃, R₄, R₅, or R₆. They can be halogen, amino, hydroxyl, mercapto, cyano, C₁∼C₆ alkyl, C₁∼C₆ alkenyl, C₁∼C₆ alkoxy, aryl, heteroaryl, or heterocycloalkyl, wherein alkyl, alkenyl, alkoxy, aryl, heteroaryl, and heterocycloalkyl are optionally substituted with C₁∼C₆ alkyl, halogen, amino, hydroxyl, mercapto, cyano. The term "alkyl" includes both linear and branched alkyl, which optionally includes one or more just-described substituted moieties.

Set forth below is an example of a fused pyrazolyl compound that can be used to practice this invention: 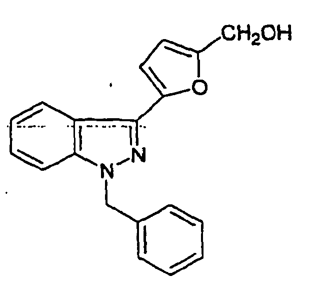 1-benzyl-3-(5'-hydroxymethyl-2'-furyl)-indazole

The fused pyrazolyl compounds described above include the compounds themselves, as well as their salts, if applicable. Such salts, for example, can be formed between a negatively charged substituent (e.g., carboxylate) on a fused pyrazolyl compound and a cation. Suitable cations include, but are not limited to, sodium ion, potassium ion, magnesium ion, calcium ion, and an ammonium cation such as teteramethylammonium ion. Likewise, a positively charged substituent (e.g., amino) can form a salt with a negatively charged counterion. Suitable counterions include, but are not limited to, chloride, bromide, iodide, sulfate, nitrate, phosphate, or acetate.

It was unexpected that the method of this invention protects neurons from cytotoxicity induced by 3-nitropropionic acid (3-NP). Other features, objects, and advantages of the invention will be apparent from the description and from the claims.

### DETAILED DESCRIPTION OF THE INVENTION

A fused pyrazolyl compound used to practice the method of this invention can be prepared by procedures well known to a skilled person in the art (see, e.g., U.S. Patent No. 5,574,168). They include the synthetic route that follows: An aryl aryl ketone is first prepared by coupling an arylcarbonyl chloride with another aryl compound. Either aryl compound is optionally mono- or multi-substituted. The ketone then reacts with an arylalkylhydrazine, the aryl group of which is also optionally mono- or multi-substituted, to form a hydrazone containing three aryl groups. The hydrazone group is transformed into a fused pyrazolyl core via an alkylene linker, another aryl group is fused at 4-C and 5-C of the pyrazolyl core, and the third aryl group is directly connected to 3-C of the pyrazolyl core. Derivatives of the fused pyrazolyl compound may be obtained by modifying the substituents on any of the aryl groups.

The chemicals used in the above-described synthetic route may include, for example, solvents, reagents, catalysts, protecting group and deprotecting group reagents. The methods described above may also additionally include steps, either before or after the steps described specifically herein, to add or remove suitable protecting groups in order to ultimately allow synthesis of the fused pyrazolyl compound. In addition, various synthetic steps may be performed in an alternate sequence or order to give the desired compounds. Synthetic chemistry transformations and protecting group methodologies (protection and deprotection) useful in synthesizing applicable fused pyrazolyl compounds are known in the art and include, for example, those described in R Larock, Comprehensive Organic Transformations, VCH Publishers (1989); T.W. Greene and P.G.M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

The fused pyrazolyl compound thus synthesized can be further purified by a method such as column chromatography, high pressure liquid chromatography, or recrystallization.

One aspect of this invention is use of the above-mentioned compounds for preparation of medicaments for treating Huntington's disease. The treatment includes administering to a subject in need thereof an effective amount of one or more fused pyrazolyl compounds and a pharmaceutically acceptable carrier. As used herein, the term "treating" refers to curing, healing, alleviating, relieving, altering, remedying, ameliorating, or preventing neurodegenerative diseases. "An effective amount" is defined as the amount of the fused pyrazolyl compound which, upon administration to a subject in need thereof, is required to confer therapeutic effect on the subject. An effective amount of the fused pyrazolyl compound can range from about 0.01 mg/kg to about 300 mg/kg. Effective doses will also vary, as recognized by those skilled in the art, depending on route of administration, excipient usage, and the possibility of co-usage with other agents for treating neurodegenerative diseases.

To implement the present invention, the fused pyrazolyl compound can be administered orally, parenterally, by inhalation spray or via an implanted reservoir. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

A composition for oral administration can be any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added. An inhalation composition can be prepared according to techniques well known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

A sterile injectable composition, for example, a sterile injectable aqueous or oleaginous suspension, can be formulated according to techniques known in the art using suitable dispersing or wetting agents (such as, for example, Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents.

A carrier in a pharmaceutical composition must be "acceptable" in the sense of being compatible with the active ingredient of the formulation (and preferably, capable of stabilizing it) and not deleterious to the subject to be treated. For example, solubilizing agents such as cyclodextrins, which form specific, more soluble complexes with the fused pyrazolyl compound, or one or more solubilizing agents, can be utilized as pharmaceutical excipients for delivery of the fused pyrazolyl compound. Examples of other carriers include colloidal silicon dioxide, magnesium stearate, cellulose, sodium lauryl sulfate, and D&C Yellow # 10.

*In vivo* screening can be performed by following procedures well known in the art. See the specific examples below.

Without further elaboration, it is believed that the above description has adequately enabled the present invention. The following specific embodiments are, therefore, to be construed as illustrative.

### CHEMICAL SYNTHESIS

Calcium borohydride was first prepared by stirring anhydrous calcium chloride (88.8 mg, 0.8 mmole) with sodium borohydride (60 mg, 1.6 mmole) in anhydrous THF (20 mL) for 4 hrs. A 30 mL THF solution containing 88.0 mg 1-benzyl-3-(5'-methoxycarbonyl-2'-furyl)-indazole (0.27 mmole) was then added dropwise to the calcium borohydride solution at 30±2 °C. The mixture was heated under reflux for 6 hrs, cooled, quenched into crushed ice, placed at a reduced pressure to remove THF, and filtered to obtain a solid product. The solid was extracted with dichloromethane. The extract was concentrated to 50 mL and a solid precipitated after petroleum ether was added. The precipitate was collected and purified by column chromatography (silica gel-benzene) to obtain 70.0 mg 1-benzyl-3-(5'-hydroxymethyl-2'-furyl)-indazole at a yield of 87%. This compound is referred to as "Indazole 1" below.
mp: 108-109°C.
MS (%), m/z: 304 (M⁺).
IR (KBr) λₘₐₓ: 3350 cm⁻¹ (-OH).
¹H-NMR (DMSO-d₆, 200 MHz) δ: 4.51 (2H, d, J=5.5 Hz, -CH₂O-), 5.31 (1H, t, J=5.5 Hz, -OH), 5.70 (2H, s, =NCH₂-), 6.48 (1H, d, J=3.4 Hz, H-4'), 6.97 (1H, d, J=3.4 Hz, H-3'), 7.21-7.31 (6H, m, H-5, phenyl), 7.45 (1H, t, J=8.2 Hz, H-6), 7.75 (1H, dd, J=8.2, 1.8 Hz, H-7), 8.12 (1H. dd, J=8.2. 1.0 Hz. C4-H).

### BIOLOGICAL ASSAYS

### Methods

*Animals:* Sprague-Dawley (SD) rats weighing 200∼300 g were used. These animals were housed in a room with controlled temperature (24±1°C) and humidity (55±5%) under a 12:12 h light-dark cycle. They were allowed free access to food and water. When surgery was performed, the rats were under anesthesia using trichloroacetaldehyde (400 mg/kg).

*Model of Huntington's disease:* Rats were injected with 3-nitropropionic acid (3-NP) (15 mg/kg/day, i.p.) for 5 successive days. In addition to examining the learning behavior using rotarod test, their brains were removed for staining of neuronal loss via cresyl violet staining, GABAergic neuron, NOS intemeuron and gliosis immunostaining.

*Immunohistochemistry:* Brain sections of various regions were isolated for the examination of dopamine (DA), glutamate decarboxylase (GAD), and a neuronal marker, e.g., glial fibrillary acidic protein (GFAP) or CD11b. The brain sections (30 µm) were incubated with the primary antibody including anti-tyrosine hydroxylase (TH, a marker of dopamine neuron), anti-neuroal-nuclei, anti-glutamate decarboxylases, anti-GFAP or anti-CD11b for 48 hr at 4°C. The brain sections were then incubated in biotinylated goat anti-rabbit IgG or anti-mouse IgG. Staining was revealed by the ABC method (Vector Laboratories, Burlingame, CA) and developed using 0.04% (w/v) diaminobenzidine to produce a brown reaction product.

*Cresyl violet staining:* Frozen sections were obtained from cryostat and mounted onto the gelatin-coated slides, dried overnight. After washing in water, the sections were stained using a 1% solution of cresyl violet acetate (Sigma) for 3 min, differentiated in 1% glacial acetic acid solution, washed with running water, and covered with Glycerin-gelatin.

*Staining of NADPH*/*d*+ *(NOS) interneurons:* NADPH diaphorase staining was performed on free-floating cryostat sections by incubating for 60 min in the dark at 37 °C in a reaction mixture containing 0.1 M Tris-HCl buffer (pH 8.0), 5 mM magnesium chloride, 2 mg/ml NADPH (reduced form; Sigma), and lmg/ml nitroblue tetrazolium (Sigma). The reaction was stopped with Tris-HCl buffer and then the sections were mounted on glass slides, dried overnight, cleared with xylene and cover-slipped with Permount.

*Rotarod Test:* Three trials were performed for each experiment in a rotarod test. For each trial, a rat was placed on a rod and timed until it fell off the rod. The test interval was 10 min. The rod was turned by an electric motor at 12 rpm.

### Results

*Prevention of 3-NP-induced neuronal death in striatum:* Rats were injected intraperitoneally with 3-NP (15 mg/kg/day) for 5 successive days. Other rats were injected concomitantly with 3-NP (15 mg/kg/day) and Indazole 1 (1 mg/kg) for 5 successive days. The brain sections of all of the rats were stained with cresyl violet. Systemic 3-NP administration produced a localized neuronal loss in the striatum. However, Indazole 1 treatment markedly reduced 3-NP-induced neuronal lesion.

To examine protection of Indazole 1 against 3-NP-induced GABAergic neurotoxicity in striatum, the brain sections were then stained with antibody against GAD. 3-NP administration caused the loss of GABAergic neuronal loss, whereas, Indazole 1 treatment protected neurons from 3-NP intoxication.

To examine protection of Indazole 1 against 3-NP-induced NADPH-d/nNOS neuronal death in striatum, the brain sections were stained by NADPH-d histochemistry to illustrate nitric oxide producing neurons. The number of survived neurons was counted from 4 brain slices per rat. It read 226±1/slice in control, 116±10/slice with injection of 3-NP, and 204±4/slice with injection of 3-NP and Indazole 1 (n=6 for each). Thus, 3-NP intoxication caused neuronal death of NADPH-d/NOS interneurons. On the other hand, Indazole 1 prevented the damage of NADPH-d/NOS interneurons.

To examine prevention of Indazole 1 against 3-NP-induced gliosis, the brain sections were then stained with antibody against GFAP, a marker of astrocyte. The result showed that chronic treatment with 3-NP caused gliosis much more severely than the concomitant treatment with 3-NP and Indazole 1.

To examine the effect of Indazole 1 on 3-NP-induced motor damage, the motor function was tested using rotarod on day 5. The rotarod was turned by an electric motor at 12 rpm. Each rat was tested thrice at an interval of 10 min and the time staying on rotarod was recorded after chronic systemic administration of 3-NP. The performance of the control rats improved with increasing numbers of trial. However, 3-NP administration severely impaired the motor learning ability. Treatment with Indazole 1 significantly improved the motor function. Time on rotarod at trial 3 was measured as 50.8±20.1 sec (n=8) in control, 2.9±0.4 sec (n=9) with injection of 3-NP, and 100.8±19.4 sec (n=13) with injection of 3-NP and Indazolel.

*Prevention of Aβ-induced neuronal death in hippocampus:* Rats received a single i.p. injection of 3-NP (20 mg/kg) dissolved in phosphate buffer and 4 hr later they received an intrahippocampal administration of Aβ fragment 25-35 (2 µg/µl) in the dentate gyrus. Day-3 after drug administration, the hippocampal region was isolated for immunohistochemical staining of a neuronal marker. Injection of Aβ in combination with single dose of 3-NP caused neuronal loss in dentate gyrus, in comparison with contralateral side. On the other hand, single intra-hippocampal injection of Indazole 1 (1.2 nmole) markedly protected neurons from Aβ-induced neuronal death.

In conclusion, Indazole 1 protects neurons from cytotoxicity induced by 3-NP. Indazole 1-related compounds are thus good candidate drugs for treating Huntington's disease.

## Claims

1. Use of a compound of the formula: 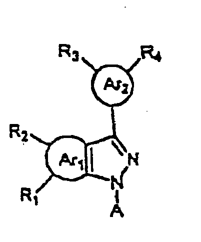 wherein A is 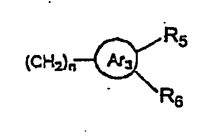 each of Ar₁ and Ar₃, is phenyl; Ar₂ is 5'-furyl;
each of R₁, R₂, R₃, R₄, R₅, and R₆, independently, is H, nitro, halogen, R, OH, OR, C(O)OH, C(O)OR, C(O)SH, C(O)SR, C(O)NH₂, C(O)NHR, C(O)NRR', ROH, ROR', RSH, RSR', ROC(O)R'OH, NHR, NRR', RNHR', or RNR'R"; or R₁ and R₂ together, R₃ and R₄ together, or R₅ and R₆ together are ORO; wherein each of R, R', and R", independently is C₁-C₆ alkyl; and
n is 1, 2, or 3
for the preparation of a therapeutic agent for the treatment of Huntington's disease.

2. The use according to claim 1, wherein n is 1.

3. The use according to claim 1 or 2, wherein each of R₁ and R₂ is H, one of R₅ and R₆ is H, and the other is Cl substituted at position 3 of phenyl or NO₂ substituted at position 4 of phenyl.

4. The use according to claim 1, wherein R₁ and R₂ are substituted at positions 4 and 5 of phenyl, respectively.

5. The use according to any one of claims 1 to 4, wherein each of R₁ and R₂ is H.

6. The use according to any one of claims 1 to 5, wherein R₁ is H and R₂ is F or OCH₃.

7. The use according to any one of claims 1 to 5, wherein R₁ and R₂ are together OCH₂O.

8. The use according to claim 1, wherein one of R₃ and R₄ is substituted at position 2 of furyl.

9. The use according to any one of claims 1 to 8, wherein one of R₃ and R₄ is H, and the other is selected from CH₂NCH₃, CH₂OH, CH₂OCH₃, COOCH₃, CH₂OH and CH₃.

10. The use according to claim 1 in which the compound is 1-benzyl-3-(5'-hydroxymethyl-2'-furyl)-indazole.

## Patentansprüche

1. Verwendung einer Verbindung der Formel 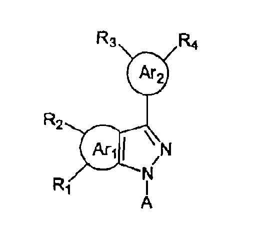 wobei 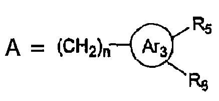 ist;
Ar₁ und Ar₃ jeweils Phenyl sind, Ar₂ = 5'-Furyl ist;
R₁, R₂, R₃, R₄, R₅ und R₆ jeweils unabhängig H, Nitro, Halogen, R, OH, OR, C(O)OH, C(O)OR, C(O)SH, C(O)SR, C(O)NH₂, C(O)NHR, C(O)NRR', ROH, ROR', RSH, RSR', ROC(O)R'OH, NHR, NRR', RNHR' oder RNR'R" sind oder R₁ und R₂ zusammen, R₃ und R₄ zusammen oder R₅ und R₆ zusammen ORO sind, wobei R, R' und R" jeweils unabhängig C₁-C₆-Alkyl sind; und
n = 1, 2 oder 3 ist;
zur Herstellung eines therapeutischen Mittels zur Behandlung von Chorea Huntington.

2. Verwendung gemäß Anspruch 1, wobei n = 1 ist.

3. Verwendung gemäß Anspruch 1 oder 2, wobei R₁ und R₂ jeweils H sind, einer der Reste R₅ und R₆ = H ist und der andere Cl, das als Substituent auf Position 3 von Phenyl gebunden ist, oder NO₂, das als Substituent auf Position 4 von Phenyl gebunden ist, ist.

4. Verwendung gemäß Anspruch 1, wobei R₁ und R₂ als Substituenten auf den Positionen 4 bzw. 5 von Phenyl gebunden sind.

5. Verwendung gemäß einem der Ansprüche 1 bis 4, wobei R₁ und R₂ jeweils H sind.

6. Verwendung gemäß einem der Ansprüche 1 bis 5, wobei R₁ = H ist und R₂ = F oder OCH₃ ist.

7. Verwendung gemäß einem der Ansprüche 1 bis 5, wobei R₁ und R₂ zusammen OCH₂O sind.

8. Verwendung gemäß Anspruch 1, wobei einer der Reste R₃ und R₄ als Substituent auf Position 2 von Furyl gebunden ist.

9. Verwendung gemäß einem der Ansprüche 1 bis 8, wobei einer der Reste R₃ und R₄ = H ist und der andere aus CH₂NCH₃, CH₂OH, CH₂OCH₃, COOCH₃, CH₂OH und CH₃ ausgewählt ist.

10. Verwendung gemäß Anspruch 1, wobei es sich bei der Verbindung um 1-Benzyl-3-(5'-hydroxymethyl-2'-furyl)indazol handelt.

## Revendications

1. Utilisation d'un composé de formule : 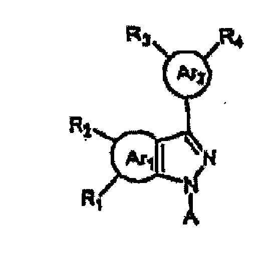 dans laquelle A est 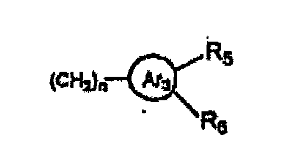 Ar₁ et Ar₃ sont chacun un phényle ; Ar₂ est un 5'-furyle ;
R₁, R₂, R₃, R₄, R₅ et R₆ sont chacun indépendamment H, un nitro, un halogène, R, OH, OR, C(O)OH, C(O)OR, C(O)SH, C(O)SR, C(O)NH₂, C(O)NHR, C(O)NRR', ROH, ROR', RSH, RSR', ROC(O)R'OH, NHR, NRR', RNHR' ou RNR'R" ; ou R₁ et R₂ ensemble, R₃ et R₄ ensemble, ou R₅ et R₆ ensemble sont ORO ; dans lesquels R, R' et R" sont chacun indépendamment un alkyle en C₁ à C₆ ; et
n est 1, 2 ou 3
pour la préparation d'un agent thérapeutique destiné au traitement de la maladie d'Huntington.

2. Utilisation selon la revendication 1, dans laquelle n est 1.

3. Utilisation selon la revendication 1 ou 2, dans laquelle R₁ et R₂ sont chacun H, un parmi R₅ et R₆ est H et l'autre est Cl en position 3 du phényle ou NO₂ en position 4 du phényle.

4. Utilisation selon la revendication 1, dans laquelle R₁ et R₂ sont respectivement en positions 4 et 5 du phényle.

5. Utilisation selon l'une quelconque des revendications 1 à 4, dans laquelle R₁ et R₂ sont chacun H.

6. Utilisation selon l'une quelconque des revendications 1 à 5, dans laquelle R₁ est H et R₂ est F ou OCH₃.

7. Utilisation selon l'une quelconque des revendications 1 à 5, dans laquelle R₁ et R₂ sont ensemble OCH₂O.

8. Utilisation selon la revendication 1, dans laquelle un parmi R₃ et R₄ est en position 2 du furyle.

9. Utilisation selon l'une quelconque des revendications 1 à 8, dans laquelle un parmi R₃ et R₄ est H et l'autre est choisi parmi CH₂NCH₃, CH₂OH, CH₂OCH₃, COOCH₃, CH₂OH et CH₃.

10. Utilisation selon la revendication 1, dans laquelle le composé est le 1-benzyl-3-(5'-hydroxyméthyl-2'-furyl)-indazole.
